# EUROPÄISCHE PATENTSCHRIFT

(11) **EP 0 677 259 B1**
(45) Veröffentlichungstag und Bekanntmachung des Hinweises auf die Patenterteilung: **21.01.1998**
(21) Anmeldenummer: 95104874.3
(22) Anmeldetag: 01.04.1995
(51) Int. Cl.: A47B 96/00, A61B 19/02, A61G 12/00

(54) **Trägerprofilplatte**
Supporting profiled panel
Panneau portant profilé

(30) Priorität: 13.04.1994 DE 4412767
(43) Veröffentlichungstag der Anmeldung: 18.10.1995
(73) Patentinhaber: Haeberle GmbH + Co. Kommanditgesellschaft, D-70565 Stuttgart (DE)
(72) Erfinder: Fuchs, Tilmann, D-70599 Stuttgart (DE)
(74) Vertreter: KOHLER SCHMID + PARTNER

(56) Entgegenhaltungen:
- EP-A- 0 072 929
- DE-A- 4 039 550
- US-A- 4 094 561
- RIWOPLAN MEDIZINISCH- TECHNISCHE EINRICHTUNGS- GESELLSCHAFT MBH, Prospekt riwo 24/1.93/D/GB "Mobile unit tower", Bundesrepublik Deutschland, Knittungen, 1993 * Gesamt *

## Beschreibung

Die vorliegende Erfindung betrifft eine Trägerprofilplatte aus Metall, insbesondere Leichtmetall, für vielfältig einsetzbare und durch verschiedene Systemkomponenten, wie z. B. Gerätehalter, Schubladen, Ablageflächen, Griffbügel usw., gestaltbare Gerätewagen zur höhenverstellbaren Befestigung der Systemkomponenten, wobei die vertikal verlaufende Trägerprofilplatte an einem über Rollen beweglichen horizontalen Basisträger befestigbar ist, wobei die zu einem Hohlkörper geformte Trägerprofilplatte eine ebene Anlagefläche aufweist, die von Öffnungen zur Durchführung von Befestigungsmitteln, z. B. Befestigungsschrauben, zur Befestigung von horizontal verlaufenden, vertikal gestaffelten Platten und Schubladen an der vertikalen Trägerprofilplatte unterbrochen ist, wobei die Seitenflächen der Systemkomponenten an der Anlagefläche der Trägerprofilplatte befestigbar sind.

Eine solche Trägerprofilplatte eines Gerätewagens ist aus dem Prospekt riwo 24/1.93/D/GB der Riwoplan Medizinisch-Technische Einrichtungsgesellschaft mbH bekannt.

Mit beweglichen Basisträgern verbundene Trägerprofilplatten werden an Gerätewagen verwendet, um die höhenverstellbare Befestigung von horizontal verlaufenden, vertikal gestaffelten Platten und Schubladen zu ermöglichen. Nach dem Zusammenbau weiter veränderbare Gerätewagen dieser Art finden in den unterschiedlichsten medizinischen Funktionsräumen, Praxen und Kliniken Verwendung und werden bevorzugt mit Spendern aller Art und Zubehör kombiniert, wodurch sich praktische Behandlungswagen und Kistenwagen ergeben. Sie werden weiter zur übersichtlichen Anordnung mehrerer Geräte innerhalb eines Raumes verwendet. Diese Anwendung beschränkt sich nicht nur auf medizinische Bereiche, sondern erfolgt ebenso in vielen technischen wie auch Bürobereichen. Die Anforderungen an einen Gerätewagen sind daher vielfältig. Die Einsatzmöglichkeiten werden weitgehend durch die Trägerprofilplatte vorgegeben, da diese die Gestaltungsmöglichkeiten des Gerätewagens festlegt.

Die in der eingangs genannten Druckschrift beschriebene Trägerprofilplatte ist mit einem U-förmigen Profil gefertigt, das zusätzliche Befestigungsteile zur Befestigung der horizontal verlaufenden, vertikal gestaffelten Platten umschließt, wodurch ein schneller Umbau des Gerätewagens erschwert wird. Nachteilig ist weiter, daß für einen Umbau bzw. eine Höhenverstellung der an der Trägerprofilplatte angebrachten Platten äußere Verkleidungsteile der Trägerprofilplatte abgeschraubt werden müssen.

Die bekannte Trägerprofilplatte kann nur in der Mitte eines horizontalen Basisträgers befestigt werden. Daher sind die Tragflächen der an der Trägerplatte angebrachten horizontal verlaufenden, vertikal gestaffelten Platten von der Seite nur eingeschränkt zugänglich, wodurch besonders das Einschieben von Geräten erschwert wird.

Falls unter den auf dem bekannten Gerätewagen untergebrachten Gegenständen elektrisch betriebene Einrichtungen sind, so hängen die Stromführungskabel sowie auch eventuelle Meß- oder Steuerleitungen außen am Wagen herunter.

Ein Kabelkanal ist bei der bekannten Trägerplatte nicht vorgesehen, so daß dieser zusätzlich am Gerätewagen angebracht werden müßte. Ein Kabelkanal könnte nur an der Rückseite des Gerätewagens angeschraubt werden, so daß diese Montage weiter die Flexibilität eines möglichen Umbaus des Gerätewagens einschränkt. Von Nachteil wäre dabei, daß ein zusätzlich befestigter Kabelkanal die freie Zugänglichkeit einer weiteren Seite, der Rückseite des Gerätewagens, einschränken würde.

Zusätzliche Zubehörteile, wie sie im medizinischen Laborbereichen benötigt werden, wie z.B. Ablageschalen, weitere Konsolen und/oder ein Kloben, können ebenfalls nicht an der Trägerplatte befestigt werden, sondern müssen an den horizontal verlaufenden, vertikal gestaffelten Platten angebracht werden, wodurch die Zugänglichkeit der Tragflächen der angebrachten Platten weiter erschwert wird. Ein beispielsweise an einer dieser Platten angebrachter einerseits vorteilhafter Kloben beeinträchtigt andererseits die Zugänglichkeit der Tragfläche zum Einschieben eines Gerätes.

Ein weiterer Nachteil der bekannten Trägerprofilplatte besteht darin, daß Flaschenaufnahmevorrichtungen, die im medizinischen Bereich benötigt werden, an der Trägerprofilplatte nur angeklemmt werden können.

Der vorliegenden Erfindung liegt die Aufgabe zugrunde, eine Trägerprofilplatte für einen multifunktionellen Gerätewagen derart weiterzuentwickeln, daß an der Trägerprofilplatte, die am horizontalen Ende der Basisträger angeordnet werden kann, Systemkomponenten, wie z.B. horizontal verlaufende, vertikal gestaffelte Platten, mit ausreichender Stabilität befestigt werden können, wodurch die Zugänglichkeit der Tragflächen der an der Trägerprofilplatte angebrachten Platten und Schubladen erhöht wird, und daß die Befestigungsabschnitte und -elemente einfach und flexibel hinter einer Sichtblende verborgen werden können.

Diese Aufgabe wird erfindungsgemäß dadurch gelöst, daß die Trägerprofilplatte einen oder mehrere Abschnitte zur Befestigung von Systemkomponenten aufweist, die im Querschnitt gesehen mehrere hintereinander parallel zur Anlagefläche angeordnete, in vertikaler Richtung verlaufende Nuten zur Aufnahme einsetzbarer Nutsteine umfassen, daß die vorderseitige Nutflanke der ersten Nut eine Öffnung zur Anlagefläche und die rückseitige Nutflanke der ersten Nut Löcher zur Durchführung von Befestigungsschrauben aufweisen, daß die weiteren Nuten durch Öffnungen in den Nutflanken miteinander verbunden sind und daß die rückseitige Nutflanke der letzten Nut eine Öffnung zur Rückseite der Trägerprofilplatte aufweist.

Die erfindungsgemäße Trägerprofilplatte hat damit den Vorteil, daß sie einstückig gefertigt werden kann und auf zusätzliche Befestigungsteile verzichtet werden kann. Durch die Befestigungsabschnitte der Trägerprofilplatte können horizontal verlaufende, vertikal gestaffelte Platten mit ausreichender Stabilität so befestigt werden, daß die Trägerprofilplatte am Ende des Gerätewagens angebracht werden kann.

Durch die Befestigungsabschnitte der Trägerplatte kann der Trägerarm bzw. die Erstreckung der horizontal verlaufenden, vertikal gestaffelten Platten, die von den Befestigungsabschnitten der Trägerprofilplatte bis zum Rand der horizontal verlaufenden Platten reicht, verlängert werden. Dies führt zu größerer auch seitlicher Zugänglichkeit der Tragflächen der horizontal verlaufenden, vertikal gestaffelten Platten, so daß Geräte leicht in die Freiräume zwischen den Platten eingeschoben werden können.

Da die Befestigungsabschnitte bei abgenommenen Dekorstreifen von hinten leicht zugänglich sind, ist der Gerätewagen leicht und schnell umbaubar ohne, daß dieser gänzlich zerlegt werden muß. Dies hat den weiteren Vorteil, daß horizontal verlaufende, vertikal gestaffelte Platten schnell, einfach und flexibel an die Trägerprofilplatte angebracht und montiert werden können.

Eine Höhenverstellung einzelner horizontal verlaufender, vertikal gestaffelter Platten ist auch jederzeit möglich.

Besonders bevorzugt ist eine Ausführungsform der erfindungsgemäßen Trägerprofilplatte, bei der an der Trägerprofilplatte ein vertikaler Kabelkanal vorgesehen ist. Die Kabel eingeschobener Geräte können so einfach und schnell verlegt werden, ohne daß ein zusätzlicher Kabelkanal montiert werden müßte, der die Zugänglichkeit einer Gerätewagenseite weiter einschränkt.

Bei einer weiteren Ausführungsform der erfindungsgemäßen Trägerprofilplatte ist der Kabelkanal mit Hilfe einer Dichtlippe verschließbar, wodurch die Kabel im Kabelkanal abgedeckt und geschützt gehalten sind.

Da die Dichtlippe bei einer Ausführungsform der Trägerprofilplatte einseitig gehalten, verschwenkbar und flexibel ist, können Kabel in jeder Höhenstufe einer horizontal verlaufenden, an der Trägerprofilplatte befestigten Platte in den verschließbaren Kabelkanal eingeführt werden.

Vorteilhafterweise kann die Dichtlippe bei einer Ausführungsform der Trägerprofilplatte aus Gummi oder flexiblem Kunststoff sein, wodurch sie einfach und billig zu fertigen ist.

Wird der Kabelkanal bei einer Ausführungsform der Trägerprofilplatte an einem der seitlichen Enden der Trägerprofilplatte positioniert, so sind die Kabel besonders einfach einzulegen und jederzeit herausnehmbar. Auch läßt sich eine durch einen seitlich angebrachten Kabelkanal erweiterte Trägerprofilplatte leicht fertigen.

Besonders bevorzugt für die Verwendung des Gerätewagens in medizinischen Laborräumen ist eine Ausführungsform der Trägerprofilplatte, bei der in vertikaler Richtung verlaufende Führungskanäle zur Aufnahme von Befestigungsrohren vorgesehen sind, die mit Befestigungsringen einfach und höhenverstellbar arretiert werden können. Dies ermöglicht eine schnelle Adaption von zusätzlichen Konsolen und Schwenkeinrichtungen, die für medizinische Anwendungen benötigt werden.

Bei einer weiteren Variante der erfindungsgemäßen Trägerprofilplatte sind an die in vertikaler Richtung verlaufenden Führungskanäle Anschlußkanäle diametral angeordnet. Die Anschlußkanäle ermöglichen auf der einen Seite eine Befestigung der Trägerprofilplatte an den horizontalen Basisträgern des Gerätewagens und können andererseits wiederum vorteilhaft zur Aufnahme von Befestigungsrohren zur Befestigung von Zubehör verwendet werden.

Eine besonders sichere Befestigung der Trägerprofilplatte an Basisträgern eines Gerätewagens läßt sich dadurch erreichen, daß bei einer Ausführungsform die Form der Anschlußkanäle im Querschnitt gesehen aus einem Kreis und an den Kreisumfang anschließenden, die Öffnung des Kreises nach außen erweiternden Umfangsegmenten gebildet ist, so daß in die Öffnung ein passendes Gegenstück eingeführt werden kann.

Bei einer weiteren Ausführungsform der Trägerprofilplatte sind an den Seitenflächen der Trägerprofilplatte Führungsnuten zur Aufnahme eines Klobens vorgesehen. Dies ist besonders bevorzugt, da ein Kloben derart befestigbar und verschwenkbar ist, daß er weitere Gegenstände greifen und halten kann. Durch die höhenverstellbare Befestigung und Verschwenkbarkeit ist der Kloben vielfältig verwendbar und behindert keine andere Verwendung des Gerätewagens. Der Kloben ermöglicht so eine stufenlose Adaption von weiterem notwendigem Zubehör.

Wenn die Befestigung von Zubehör durch Befestigungsrohre und den Kloben nicht notwendig ist, ist es vorteilhaft, daß die Öffnungen des oberen Endes der Trägerprofilplatte, die Befestigungsrohre und Befestigungskloben aufnehmen, mit einer Abdeckung formschlüssig abschließbar sind. Durch die genaue Passform der Abdeckung, die dem Querschnittsprofil der Trägerprofilplatte entspricht, kann die Abdeckung schnell und sicher erfolgen.

Bei einer Ausführungsform der Trägerprofilplatte kann in die Nut ein Dekorstreifen zur Abdeckung der Befestigungsschrauben und Nutsteine eingesetzt werden. Dadurch werden die Befestigungsabschnitte abgedeckt, geschützt und gleichzeitig schließt der Dekorstreifen die Rückseite formschlüssig ab, wodurch die Rückseite der Trägerprofilplatte keine scharfen Ecken und Kanten aufweist, die eine Verletzungsgefahr beinhalten könnten.

Bevorzugt kann der Dekorstreifen farbig gestaltet sein, wodurch eine Kennzeichnung des Gerätewagens für verschiedene Benutzungszwecke möglich ist.

Weitere Vorteile der Erfindung ergeben sich aus der nachfolgenden Beschreibung von Ausführungsbeispielen der Erfindung anhand der Zeichnung. Es zeigen:
- Fig. 1: eine schematische Schnittdarstellung einer Trägerprofilplatte;
- Fig. 2: eine schematische Schnittdarstellung eines Befestigungsabschnitts der Trägerprofilplatte;
- Fig. 3: eine schematische Schnittdarstellung einer weiteren Ausführungsform der Trägerprofilplatte;
- Fig. 4: eine schematische Schnittdarstellung einer weiteren Ausführungsform der Trägerprofilplatte; und
- Fig. 5: eine schematische Schnittdarstellung einer weiteren Ausführungsform der Trägerprofilplatte; und
- Fig. 6: einen zusammengebauten Gerätewagen mit einer seitlich angebrachten Trägerprofilplatte.

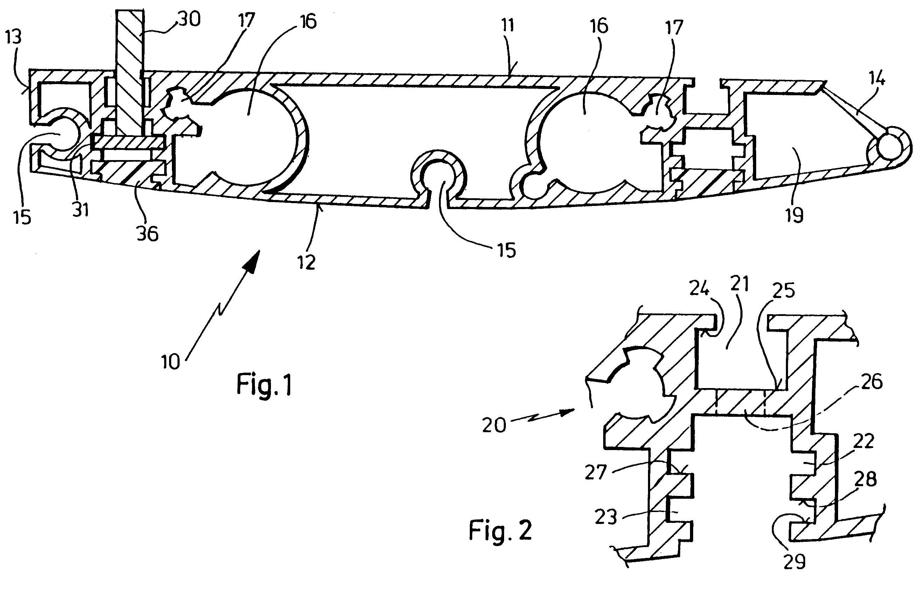

Fig. 1 zeigt eine schematische Schnittdarstellung der Trägerprofilplatte 10, die eine mögliche Kombination von Merkmalen der erfindungsgemäßen Trägerprofilplatte aufweist.

Die Trägerprofilplatte 10 umschließt mit der ebenen Anlagefläche 11, der Rückseite 12 und der Seitenfläche 13 und der Dichtlippe 14 einen Hohlkörper.

An der Seitenfläche 13 ist beispielhaft eine Führungsnut 15 angebracht, die zur Aufnahme eines Klobens dient. Ein Kloben kann in die Führungsnut 15 von oben eingeschoben werden, so daß der Kloben höhenverstellbar in der Führunngsnut 15 befestigt werden kann. Auch an der Rückseite 12 ist eine solche Führungsnut 15 angeformt. Die Führungsnuten 15 sind nach außen offen. Sie können aber auch geschlossen gebildet sein.

In die Führungskanäle 16 können Befestigungsrohre in der Höhe verstellbar von oben eingesetzt und mit Befestigungsringen arretiert werden.

An die Führungskanäle 16 schließen sich weitere Kanäle 17 diametral an, die zur Befestigung der Trägerprofilplatte 10 an dem horizontalen Basisträger 18 dienen. Gleichzeitig kann wiederum auch in die Kanäle 17 ein Befestigungsrohr eingesetzt werden, das mit einem Befestigungsring arretiert werden kann und an dem weitere Zubehörteile befestigt werden können.

Seitlich ist an der Trägerprofilplatte ein Kabelkanal 19 angeordnet, der über die Dichtlippe 14 verschließbar ist.

Durch zwei Befestigungsabschnitte 20 können an der Trägerprofilplatte 10 horizontal verlaufende Platten befestigt werden.

Fig. 2 zeigt eine schematische Schnittdarstellung eines Befestigungsabschnittes 20. Mehrere hintereinander gestaffelt angeordnete Nuten 21, 22 und 23 verlaufen parallel zur Anlagefläche 11. Die erste Nut 21 zur Anlagefläche 11 hin weist eine Öffnung in ihrer Nutflanke 24 und ein Loch in ihrer Nutflanke 25 auf. Die Nuten 22 und 23 sind durch Öffnungen in ihren Nutflanken 27 und 28 miteinander verbunden. Da die Nutflanke 29 der Nut 23 eine Öffnung zur Rückseite 12 der Trägerprofilplatte 10 besitzt, kann eine Befestigungsschraube von der Rückseite 12 der Trägerprofilplatte 10 durch die Öffnungen in das Loch 26 der Nutflanke 25 der Nut 21 eingesetzt werden und mit einem in die Nut 22 eingesetzten Nutstein befestigt werden, so daß eine horizontal verlaufende Platte an der Anlagefläche 11 der Trägerprofilplatte 10 befestigt wird.

In die Nut 23 ist ein Dekorstreifen 36 einsetzbar, der die Rückseite der Trägerprofilplatte formschlüssig abschließt und die Befestigungsabschnitte der Trägerprofilplatte abdeckt, so daß sie nach außen nicht sichtbar sind. Ebenso kann auch der nach oben offene Querschnitt mit einer Abdeckung versehen werden, wenn keine Befestigungsrohre für Zubehör benötigt werden.

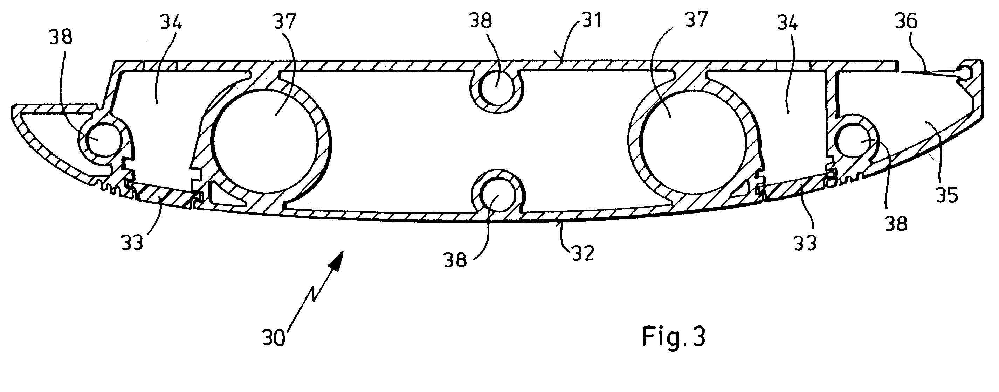

Weitere Ausführungsformen einer Trägerprofilplatte sind in den Figuren 3 bis 5 gezeigt, die aus Änderungen des Querschnittsprofils der Fig. 1 hervorgehen. Die Figuren 3 bis 5 demonstrieren beispielhaft veränderte Anordnungsmöglichkeiten der Führungskanäle und Führungensnuten zur Aufnahme der Befestigungsrohre und Befestigungskloben, die einen vielfältigen Einsatz der Trägerprofilplatte und des Gerätewagens gewähren.

Fig. 3 zeigt eine Trägerprofilplatte 30 mit einer Anlagefläche 31 und einer Rückseite 32. Nutsteine 33 schließen die nur angedeuteten Befestigungsabschnitte 34 formschlüssig mit der Rückseite 32 ab. Seitlich ist ein Kabelkanal 35 angeordnet, der eine Öffnung in der durch die Anlagefläche 31 definierten Ebene aufweist, die durch eine Dichtlippe 36 verschließbar ist. In die Führugsnuten 38 können wiederum Kloben eingesetzt werden.

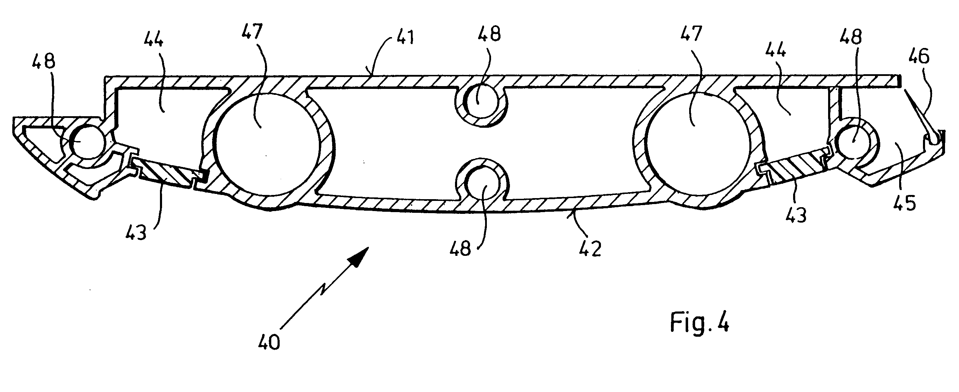

Fig. 4 zeigt eine Trägerprofilplatte 40 mit einer Anlagefläche 41 und einer Rückseite 42. Nutsteine 43 schließen die auch hier nur angedeuteten Befestigungsabschnitte 44 formschlüssig mit der Rückseite 42 ab. Seitlich ist ein Kabelkanal 45 angeordnet, der eine Öffnung zur Seite hin aufweist, die durch eine Dichtlippe 46 verschließbar ist. In die Führugsnuten 48 können wiederum Kloben eingesetzt werden.

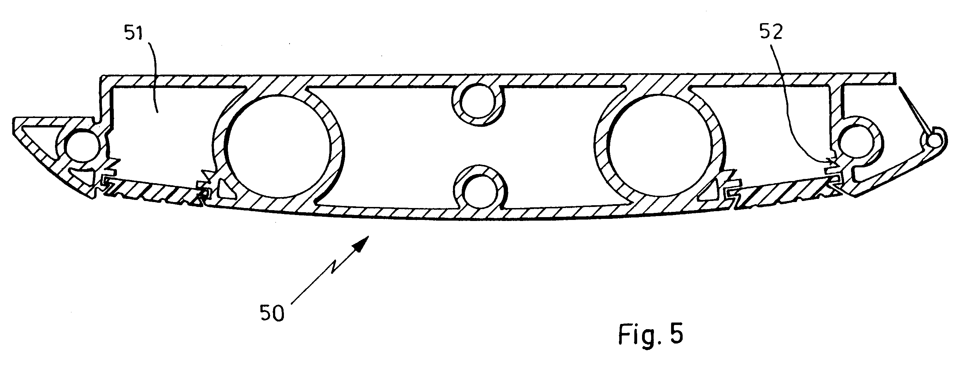

Fig. 5 zeigt eine Trägerprofilplatte 50, die im wesentlichen der Trägerprofilplatte 40 der Fig. 4 entspricht, wobei die auch hier nur angedeuteten Befestigungsabschnitte 51 Nuten 52 aufweisen, deren Querschnittsform von einer rechteckigen Querschnittsform verschieden ist.

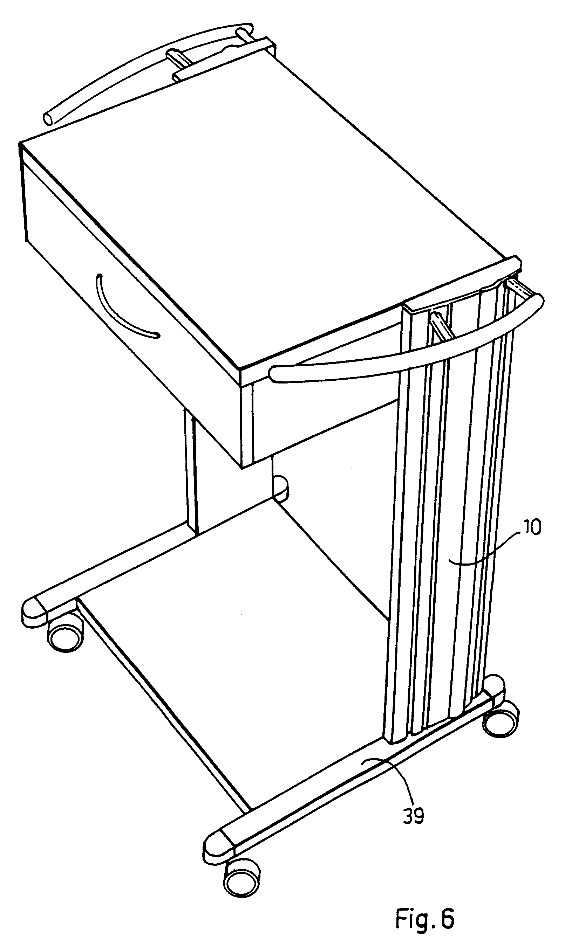

Figur 6 zeigt einen zusammengebauten Gerätewagen mit einer seitlich angeordneten Trägerprofilplatte 10, die am Basisträger 39 festigt ist.

## Patentansprüche

1. Trägerprofilplatte (10) aus Metall, insbesondere Leichtmetall, für vielfältig einsetzbare und durch verschiedene Systemkomponenten, wie z.B. Gerätehalter, Schubladen, Ablageflächen, Griffbügel usw., gestaltbare Gerätewagen zur höhenverstellbaren Befestigung der Systemkomponenten,
wobei die vertikal verlaufende Trägerprofilplatte (10) an einem über Rollen beweglichen horizontalen Basisträger (39) befestigbar ist,
wobei die zu einem Hohlkörper geformte Trägerprofilplatte (10) eine ebene Anlagefläche (11) aufweist, die von Öffnungen zur Durchführung von Befestigungsmitteln, z.B. Befestigungsschrauben, zur Befestigung von horizontal verlaufenden, vertikal gestaffelten Platten und Schubladen an der vertikalen Trägerprofilplatte (10) unterbrochen ist,
und wobei die Seitenflächen der Systemkomponenten an der Anlagefläche (11) der Trägerprofilplatte (10) befestigbar sind,
dadurch gekennzeichnet,
daß die Trägerprofilplatte (10) einen oder mehrere Abschnitte (20) zur Befestigung von Systemkomponenten aufweist, die im Querschnitt gesehen mehrere hintereinander parallel zur Anlagefläche (11) angeordnete, in vertikaler Richtung verlaufende Nuten (21, 22, 23) zur Aufnahme einsetzbarer Nutsteine (31) umfassen,
daß die vorderseitige Nutflanke (24) der ersten Nut (21) eine Öffnung zur Anlagefläche (11) und die rückseitige Nutflanke (25) der ersten Nut (21) Löcher (26) zur Durchführung von Befestigungsschrauben (30) aufweisen,
daß die weiteren Nuten (22, 23) durch Öffnungen in den Nutflanken (27, 28) miteinander verbunden sind, und
daß die rückseitige Nutflanke (29) der letzten Nut (23) eine Öffnung zur Rückseite (12) der Trägerprofilplatte (10) aufweist.

2. Trägerprofilplatte nach Anspruch 1, dadurch gekennzeichnet, daß an der Trägerprofilplatte (10) ein vertikaler Kabelkanal (19) vorgesehen ist.

3. Trägerprofilplatte nach Anspruch 2, dadurch gekennzeichnet daß der Kabelkanal (19) mit Hilfe einer Dichtlippe (14) verschließbar ist.

4. Trägerprofilplatte nach Anspruch 3, dadurch gekennzeichnet, daß die Dichtlippe (14) einseitig gehalten, verschwenkbar und flexibel ist.

5. Trägerprofilplatte nach einem der Ansprüche 2 bis 4, dadurch gekennzeichnet, daß der Kabelkanal (19) an einem der seitlichen Enden der Trägerprofilplatte (10) positioniert ist.

6. Trägerprofilplatte nach einem der vorherigen Ansprüche, dadurch gekennzeichnet, daß im Innern der Trägerprofilplatte (10) in vertikaler Richtung verlaufende Führungskanäle (16) zur Aufnahme von Befestigungsrohren vorgesehen sind.

7. Trägerprofilplatte nach Anspruch 6, dadurch gekennzeichnet, daß zu den in vertikaler Richtung verlaufenden Führungskanälen (16) Anschlußkanäle (17) diametral angeordnet sind.

8. Trägerprofilplatte nach Anspruch 7, dadurch gekennzeichnet, daß die Form der Anschlußkanäle (17) im Querschnitt gesehen aus einem Kreis und an den Kreisumfang anschließenden, die Öffnung des Kreises nach außen erweiternden Umfangssegmenten gebildet ist.

9. Trägerprofilplatte nach einem der vorherigen Ansprüche, dadurch gekennzeichnet, daß an Seitenblechen der Trägerprofilplatte (10) Führungsnuten (15) zur Aufnahme eines Klobens vorgesehen sind.

10. Trägerprofilplatte nach einem der vorherigen Ansprüche, dadurch gekennzeichnet, daß die Öffnungen des oberen Endes der Trägerprofilplatte, die Befestigungrohre und Befestigungskloben aufnehmen, durch eine Abdeckung formschlüssig abschließbar sind.

## Claims

1. Profiled support plate (10) made from metal, in particular made from light weight metal, for tool wagons having a plurality of applications and with differing system components such as e.g. apparatus supports, drawers, seating surfaces, handles and the like, for the mounting of the system components in a vertically adjustable fashion,
whereby the vertically extending profiled support plate (10) can be attached to a horizontal base support (39) which is movable by means of rollers,
whereby the hollow-body profiled support plate (10) has a flat seating surface (11) which is interrupted by openings for passage of mounting means, e.g. mounting screws, to mount horizontally extending vertically stacked plates and drawers onto the vertical profiled support plate (10),
and whereby the side surfaces of the system components can be mounted on the seating surface (11) of the profiled support plate (10),
characterized in that,
the profiled support plate (10) has one or more sections (20) for attachment of system components which, as viewed in cross section, include a plurality of grooves (21, 22, 23) disposed parallel to the seating surface (11) behind each other and extending in a vertical direction for accepting sliding blocks (31) which can be inserted therein,
and the front side (24) of the first groove (21) has an opening towards the seating surface (11) and the back side (25) of the first groove (21) has holes (26) for the passage of mountings screws (30),
and the additional grooves (22, 23) are connected to each other by means of openings in the sides of the grooves (27, 28), and
the back side (29) of the last groove (23) has an opening towards the back side (12) of the profiled support plate (10).

2. Profiled support plate according to claim 1, characterized in that a vertical cable channel (19) is provided for on the profiled support plate (10).

3. Profiled support plate according to claim 2, characterized in that the cable channel (19) can be closed with the assistance of a sealing lip (14).

4. Profiled support plate according to claim 3, characterized in that the sealing lip (14) is supported at one side, is pivotable, and is flexible.

5. Profiled support plate according to any one of the claims 2 through 4, characterized in that the cable channel (19) is disposed on one of the sideward ends of the profiled support plate (10).

6. Profiled support plate according to any one of the previous claims, characterized in that vertically extending guide channels (16) are provided inside the profiled support plate (10) for the acceptance of mounting pipes.

7. Profiled support plate according to claim 6, characterized in that connector channels (17) are diametrically disposed across from the vertically extending guide channels (16).

8. Profiled support plate according to claim 7, characterized in that the connector channels (17), as viewed in cross section, have the shape of a circle with peripheral segments adjacent to the periphery of the circle which extend the opening of the circle in the outer direction.

9. Profiled support plate according to any one of the preceding claims, characterized in that guide grooves (15) are provided on the sides of the profiled support plate (10) for acceptance of a clamp block.

10. Profiled support plate according to any one of the preceding claims, characterized in that the openings of the upper end of the profiled support plate which accept the mounting pipes and the clamp blocks can be smoothly closed by a cover.

## Revendications

1. Panneau portant profilé (10) en métal, en particulier en métal léger, destiné à des chariots susceptibles d'être utilisés de façon multiple et d'être équipés de composants différents d'un système, tels que par exemple des porte-appareils, des tiroirs, des surfaces d'appui, des anses de saisie, etc., pour la fixation réglable en hauteur des composants d'un système,
dans lequel le panneau portant profilé (10) qui s'étend verticalement est susceptible d'être fixé sur un élément porteur de base (39) horizontalement mobile au moyen de galets,
et le panneau portant profilé (10), formé à la manière d'un corps creux, présente une surface d'appui plane (11) qui est interrompue par des ouvertures destinées à la traversée d'organes de fixation, par exemple des vis de fixation, destinés à la fixation de plaques et de tiroirs horizontaux étagés verticalement, sur le panneau portant profilé vertical (10),
et les surfaces latérales des composants du système peuvent être fixées sur la surface d'appui (11) du panneau portant profilé (10),
caractérisé en ce que
le panneau portant profilé (10) comporte un ou plusieurs tronçons (29) pour la fixation de composants d'un système, lesdits tronçons présentant, vus en section transversale, plusieurs gorges (21, 22, 23) agencées les unes derrière les autres parallèlement à la surface d'appui (11) et s'étendant en direction verticale, pour recevoir des patins (31) à mettre en place,
le flanc côté avant (24) de la première gorge (21) présente une ouverture vers la surface d'appui (11) et le flanc arrière (25) de la première gorge (21) présente des trous (26) pour la traversée de vis de fixation (30),
les autres gorges (22, 23) sont reliées les unes aux autres via des ouvertures dans les flancs (27, 28) des gorges,
et le flanc arrière (29) de la dernière gorge (23) présente une ouverture vers le côté arrière (12) du panneau portant profilé (10).

2. Panneau portant profilé selon la revendication 1, caractérisé en ce que sur le panneau portant profilé (10) est prévu un canal à câbles vertical (19).

3. Panneau portant profilé selon la revendication 2, caractérisé en ce que le canal à câbles (19) peut être obturé à l'aide d'une lèvre d'étanchéité (14).

4. Panneau portant profilé selon la revendication 3, caractérisé en ce que la lèvre d'étanchéité (14) est tenue d'un côté, capable de basculer et flexible.

5. Panneau portant profilé selon l'une des revendications 2 à 4, caractérisé en ce que le canal à câbles (19) est positionné à l'une des extrémités latérales du panneau portant profilé (10).

6. Panneau portant profilé selon l'une des revendications précédentes, caractérisé en ce qu'à l'intérieur du panneau portant profilé (10) sont prévus des canaux de guidage verticaux (16) pour recevoir des tubes de fixation.

7. Panneau portant profilé selon la revendication 6, caractérisé en ce que des canaux de raccordement (17) sont agencés diamétralement par rapport aux canaux de guidage (16) qui s'étendent verticalement.

8. Panneau portant profilé selon la revendication 7, caractérisé en ce que la forme des canaux de raccordement (17), vue en coupe transversale, est formée par un cercle et par des segments périphériques qui se raccordent à la périphérie du cercle et qui élargissent l'ouverture du cercle vers l'extérieur.

9. Panneau portant profilé selon l'une des revendications précédentes, caractérisé en ce que des gorges de guidage (15) sont prévues sur les tôles latérales du panneau portant profilé (10) pour recevoir un bloc.

10. Panneau portant profilé selon l'une des revendications précédentes, caractérisé en ce que les ouvertures de l'extrémité supérieure du panneau portant profilé, qui reçoivent les tubes de fixation et les blocs de fixation, peuvent être refermées en coopération de formes par un couvercle.
